# EUROPEAN PATENT SPECIFICATION

(11) **EP 2 598 961 B1**
(45) Date of publication and mention of the grant of the patent: **02.09.2020**
(21) Application number: 11743723.6
(22) Date of filing: 28.07.2011
(51) Int. Cl.: G05B 19/042

(54) **HANDHELD FIELD MAINTENANCE TOOL WITH FIELD DEVICE SIMULATION CAPABILITY**
TRAGBARES WARTUNGSWERKZEUG MIT FELDGERÄTESIMULATIONSFÄHIGKEIT
OUTIL DE MAINTENANCE PORTATIF AVEC CAPACITÉ DE SIMULATION DE DISPOSITIF DE TERRAIN

(30) Priority: 28.07.2010 US 368477 P
(43) Date of publication of application: 05.06.2013
(73) Proprietor: Fisher-Rosemount Systems, Inc., Austin, Texas 78759 (US)
(72) Inventor: KANTZES, Christopher, P., Minneapolis MN 55419 (US); MATHIOWETZ, Brad, N., Lakeville MN 55044 (US); TOEPKE, Todd, M., Eden Prairie MN 55347 (US); YANG, Kun, Eden Prairie MN 55347 (US); LUND, Adam, E., St. Louis Park MN 55426 (US)
(74) Representative: Bohnenberger, Johannes
(86) International application number: PCT/US2011/045680
(87) International publication number: WO 2012/016013

(56) References cited:
- EP-A1- 1 916 582
- WO-A1-2009/074544
- DE-A1- 10 245 176
- DE-A1-102009 028 195
- JP-A- H07 154 879
- US-A1- 2004 193 287
- US-A1- 2006 161 393
- US-A1- 2010 114 347

## Description

### BACKGROUND

Handheld field maintenance tools are known. Such tools are highly useful in the process control and measurement industry to allow operators to conveniently communicate with and/or interrogate field devices in a given process installation. Examples of such process installations include petroleum, pharmaceutical, chemical, pulp, and other fluid processing installations. In such installations, the process control and measurement network may include tens or even hundreds of various field devices which periodically require maintenance to ensure that such devices are functioning properly and/or calibrated. Moreover, when one or more errors in the process control and measurement installation are detected, the use of a handheld field maintenance tool allows a technician to quickly diagnose such errors in the field. Handheld field maintenance tools are generally used to configure, calibrate, and diagnose problems relative to intelligent field devices using digital process communication protocols.

Since at least some process installations may involve highly volatile, or even explosive, environments, it is often beneficial, or even required, for field devices and the handheld field maintenance tools used with such field devices to comply with intrinsic safety requirements. These requirements help ensure that compliant electrical devices will not generate a source of ignition even under fault conditions. One example of Intrinsic Safety requirements is set forth in: APPROVAL STANDARD INTRINSICALLY SAFE APPARATUS AND ASSOCIATED APPARATUS FOR USE IN CLASS I, II and III, DIVISION NUMBER 1 HAZARDOUS (CLASSIFIED) LOCATIONS, CLASS NUMBER 3610, promulgated by Factory Mutual Research October, 1998. An example of a handheld field maintenance tool that complies with intrinsic safety requirements includes that sold under trade designation Model 475 Field Communicator, available from Emerson Process Management of Austin, Texas. Patent Literature US 2004/0193287 A1 relates to a method for the offline parametering of a field device for process automation technology with the help of an operating program B running on an operating device B, comprising the steps of communicating an operating program with a field device over a data bus for online parametering and for which no device description is available describing the offline behavior of the field device F1; and communicating the operating program B with a copy of the device software program GS running in the field device, thereby simulating an online field device.

### SUMMARY

According to the invention, the problem is solved by means of a handheld field maintenance tool, and a method for simulating a field device over a process communication loop, as defined in the independent claims. Advantageous further developments of the invention are set forth in the dependent claims.

### BRIEF DESCRIPTION OF THE DRAWINGS

FIGS. 1A and 1B are diagrammatic views of a handheld field maintenance tool with which embodiments of the invention are particularly useful.
FIG. 2 is a diagrammatic view of a handheld field maintenance tool with which embodiments of the present invention are particularly useful.
Fig. 3 is a block diagram of a handheld field maintenance tool in accordance with an embodiment of the present invention.
FIG. 4 is a flow diagram of a method of simulating a field device in accordance with an embodiment of the present invention.

### DETAILED DESCRIPTION

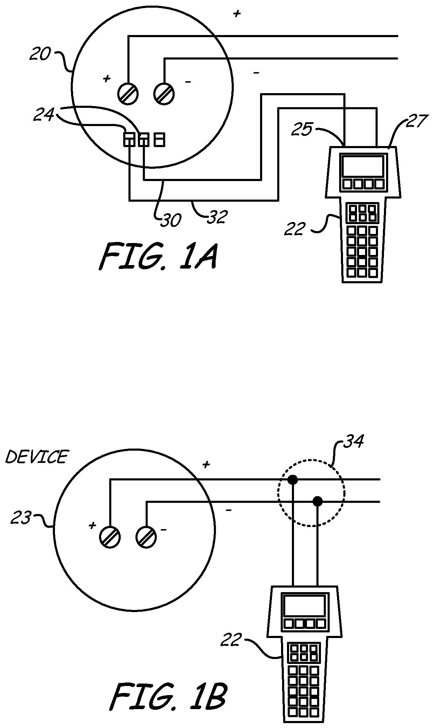

FIGS. 1A and 1B are diagrammatic views of a handheld field maintenance tool 22 coupled to field devices 20, 23. As shown in FIG. 1A, handheld field maintenance tool 22 includes a pair of terminals 25, 27 that couple to test leads 30, 32, respectively, which are then coupled to terminals 24 of field device 20. Terminals 24 may be dedicated terminals to allow such a handheld field maintenance tool to couple to device 20 and interact with device 20. The utilization of terminals 25, 27 to couple to field device illustrates an example of a wired connection between handheld field maintenance tool 22 and field device 20.

FIG. 1B shows an alternate arrangement where handheld field maintenance tool 22 couples directly to the process control loop 34 to which field device 23 is coupled. In either case, the wired connection between the handheld field maintenance tool and the field device allows the handheld field maintenance tool to interact with the desired field device 20, 23.

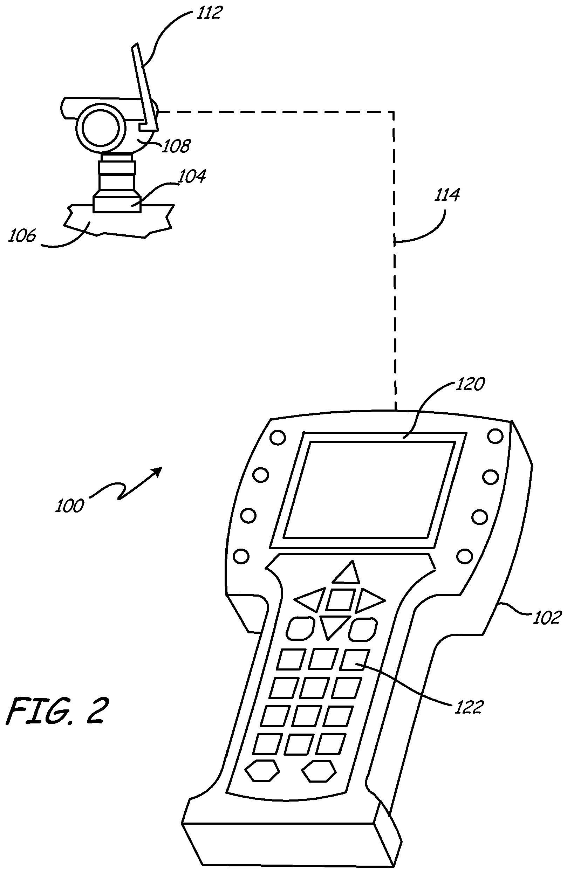

FIG. 2 is a diagrammatic view of handheld field maintenance tool 102 interacting with wireless field device 104. System 100 includes handheld field maintenance tool 102 communicating with field device 104. Handheld field maintenance tool 102 is communicatively coupled to field device 104 via communication link 114. Communication link 114 can take any suitable form including wired connections as shown in FIGS. 1A and 1B, as well as wireless communication techniques that are currently being used or being developed. Handheld field maintenance tool 102 allows a technician to interact with field device 104 to configure, calibrate, and/or diagnose problems with respect to field device 104 using a digital process communication protocol such as FOUNDATION™ Fieldbus and/or the HART® protocol. Handheld field maintenance tools, such as tool 102 can be used to save configuration data from field devices, such as field device 104.

Field device 104 may be any device that senses a variable in the process and transmits information related to the variable over a process communication loop; such as a pressure or temperature. Field device 104 may also be a device that receives information from a process communication loop and sets a physical parameter, such as a valve closure, based on the information. Field device 104 is depicted as an industrial process fluid pressure transmitter having a pressure manifold 106 coupled thereto, and an electronics enclosure 108. Field device 104 is provided for illustrative purposes only. In reality, field device 104 may be any industrial device, such as a process fluid temperature transmitter, process fluid level transmitter, process fluid flow transmitter, valve controller, or any other device that is useful in the measurement and/or control of industrial processes.

Handheld field maintenance tool 102 generally includes a user interface that comprises a display 120 as well as a number of user input buttons 122. Display 120 may be any suitable display such as an active-matrix liquid crystal display, or any other suitable display that is able to provide useful information. Buttons 122 may comprise any suitable arrangement of buttons relative to any number of functions to which the handheld field maintenance tool may be directed. Buttons 122 may comprise a numeric keypad, an alphanumeric keypad, any suitable number of custom functions and/or navigation buttons, or any combination thereof.

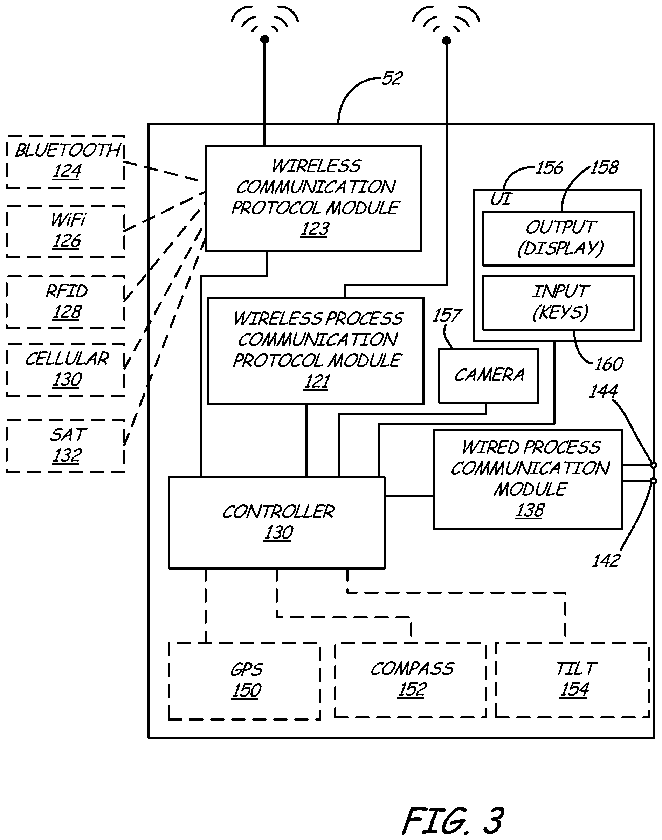

Fig. 3 is a diagrammatic system block diagram of a handheld field maintenance tool in accordance with the embodiment of the present invention. It is preferred that tool 52 comply with at least one intrinsic safety specification, such as that listed above, in order to help ensure safety in potentially explosive environments. Handheld field maintenance tool 52 includes at least one wireless process communication module 121. Suitable examples for wireless process communication module 121 include a module that generates and/or receives proper signals in accordance with a known wireless communication protocol, such as the known WirelessHART protocol (IEC 62591). Another wireless process communication protocol is set forth in ISA100.11a. While Fig. 3 shows a single wireless process communication module 121, it is expressly contemplated that any suitable number of wireless process communication modules can be used to communicate in accordance with various wireless process communication protocols now in existence or later developed.

Handheld field maintenance tool 52 also includes at least one secondary wireless communication protocol module 123. Wireless communication protocol module 123 can communicate in accordance with one or more of the options shown in phantom in Fig. 3. Specifically, wireless communication protocol module 123 may communicate in accordance with a Bluetooth specification 124 (such as Bluetooth Specification 2.1 rated at Power Class 2; a Wi-Fi specification 126 (such as IEEE 802.11.a/b/g/n); a known RFID specification 128; cellular communication techniques 130 (such as GSM/CDMA); and/or satellite communication 132. These communication techniques and methodologies allow handheld field maintenance tool 52 to communicate directly with a wireless gateway or other suitable device either via direct wireless communication, or using the Internet. While one wireless communication protocol module 123 is shown in Fig. 3, any suitable number may be used. Each of the wireless process communication protocol module 121 and wireless communication protocol module 123 is coupled to controller 130 which is also coupled to the wired process communication module 138. Controller 130 is preferably a microprocessor that executes a sequence of instructions stored therein, or in memory coupled to controller 130, to perform handheld field maintenance tasks. Wired process communication module 138 allows handheld field maintenance tool 52 to be physically coupled via a wired connection at terminals 142, 144 to a field device. Examples of suitable wired process communication include the Highway Addressable Remote Transducer (HART®) protocol, the FOUNDATION™ Fieldbus protocol, Profibus and others.

Handheld field maintenance tool 52 includes a user interface module 156 for generating a user interface using display 120 and keys 122. Module 156 can include suitable display driver circuitry 158 and/or memory to interact with display 120. Module 156 also includes input circuitry 160 which is configured to interact with buttons 122 to receive user input. Additionally, in embodiments where display 120 includes a touchscreen, module 160 can include circuitry to generate user input data to controller 130 based upon a user's touch and/or gestures received by the touchscreen.

Handheld field maintenance tool 52 can include a number of additional items that facilitate additional functionality. Specifically, tool 52 can include a position detection module, such as GPS module 150. GPS module 150 can be configured to additionally use the Wide Area Augmentation System (WAAS) for improved accuracy and/or can be configured to operate using differential GPS techniques as appropriate. Module 150 is coupled to controller 130 to provide controller 130 with an indication of the geographic position of tool 52. While position detection module 150 is preferably an internal component of tool 52, it may be external and communicatively coupled thereto using a suitable wireless or wired communication protocol, such as Bluetooth 124, RFID 128, et cetera. Further still, while position detection module 150 is generally described as GPS module 150, other techniques for triangulating the position of the handheld field maintenance tool based upon relative strength of wireless communication with wireless transceivers having known fixed positions can be employed. Examples of such wireless triangulation techniques include triangulation of the position of handheld field maintenance tool 52 based upon communication with three or more fixed-position WiFi communication points, or access points. Further still, as set forth above, embodiments of the present invention may include the ability to employ one or more wireless process communication protocol modules, such as module 121. Such triangulation techniques can also be employed if a suitable number of wireless interactions with fixed-position wireless field devices can be achieved. Finally, while the various methods provided for obtaining the position of handheld field maintenance tool 52 are described above, they can also be used in conjunction with one another to provide additional accuracy and/or redundancy. Additionally, tool 52 also preferably comprises compass module 152 coupled to controller 130 such that tool 52 can indicate the compass direction in which it is pointing. Finally, tool 52 can also include tilt module 154 coupled to controller 130 to provide an indication to controller 130 relative to an angle of inclination of tool 52 relative to gravity. However, additional axes of sensing are also contemplated.

The positional location module 150, compass module 152 and tilt module 154 are particularly useful where a handheld field maintenance tool helps a technician or engineer find the physical location of a wireless field device in the field. An oil refinery is often a very large process installation with many field devices positioned at various locations, some of which may not be readily visible

Setting up a process control system often requires that the control strategy be verified by forcing various field devices to simulate a response signal in order to observe the control system response and determine if it is correct. This currently requires that the actual field device instrument be installed and powered. Once those criteria are established, the field device(s) must then be manipulated so that the field device(s) provides or otherwise outputs the desired digital signal. This can be done by placing the field device in a simulate mode and using a configuration device, such as a handheld field maintenance tool, to set the desired output. Alternatively, the desired output can be obtained by actually applying an external source to the sensor(s) of the field device(s). For example, for a process fluid pressure transmitter, a specific pressure can be applied. The simulation capability is not always available in all field devices. Moreover, even if a simulation capability is provided, it can sometimes be difficult or cumbersome to use. Further still, the external source solution, on the other hand, can be very time consuming and cumbersome to implement.

In accordance with an embodiment of the present invention, a handheld field maintenance tool is provided with an ability to actually mimic one or more field devices on a process control loop or segment. In this manner, the handheld field maintenance tool behaves as if it were an actual field device or devices. This means that the control setup can now be verified ahead of any instrumentation being physically installed in the field. Moreover, embodiments of the present invention can provide a simulation interface that is optimized for ease of use for the technician and can allow the technician to select specific field devices to simulate through the use of device description (DD) technology. In addition, embodiments of the present invention can specify what the configuration of the simulated field device should be, and what the dynamic variable output should be.

In accordance with an embodiment of the present invention, handheld field maintenance tool 52 includes a database of device descriptions stored within memory of controller 130, or memory coupled to controller 130. Additionally, or alternatively, any suitable device description can be obtained from a remote device via wireless communication protocol module 123.

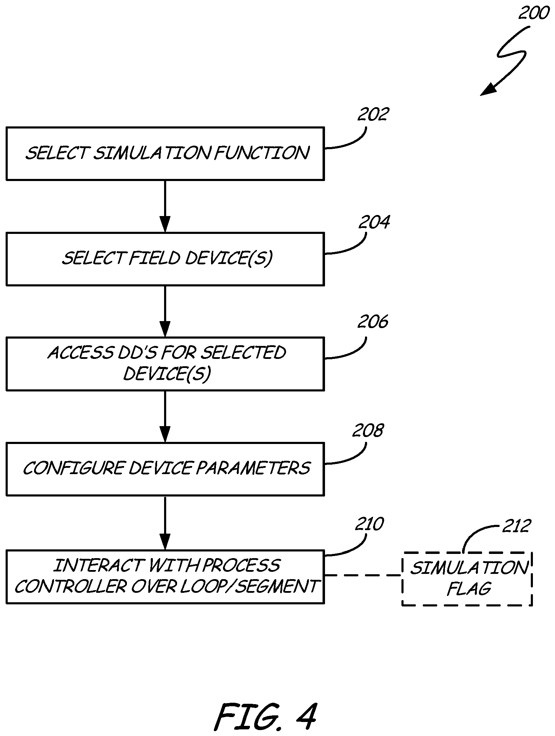

FIG. 4 is a flow diagram of a method of simulating a field device in accordance with an embodiment of the present invention. Method 200 begins at block 202 where a technician or user of a handheld field maintenance tool, such as tool 52, selects the device simulation function. This selection can be performed either through pressing an appropriate button 122, or via navigation of a handheld field maintenance device menu. Once the device simulation function has been selected, controller 130 causes the display 120 to provide one or more user interface elements that help the user select a particular field device. For example, the user interface may include a dropdown box that lists all known field device manufacturers. Then, once a user selects a device manufacturer, a second user interface element may provide the selection of a device type. Once the device type has been selected, a third user interface element may provide a comprehensive listing of all known field devices manufactured by the selected manufacturer of the selected type. Once the user selects a specific field device(s) at block 204, a device description (DD) is accessed for the selected field device(s), as indicated at block 206. Additionally, if the user indicates that the field device is not provided in the list of field devices presented to the user from the selected manufacturer of the selected type, method 200 can include the ability of a handheld field maintenance tool to access an online database of field devices available from the selected manufacturer of the selected type using wireless communication protocol module 123. At block 206, access of the requisite device description(s) is preferably accomplished via an internal lookup of a database of device descriptions stored locally within a handheld field maintenance tool. If the required device description(s) is not stored within a local device description database, or if no database is provided, handheld field maintenance tool 52 can access the requisite device description(s) preferably via wireless communication protocol module 123 over the Internet, or any suitable network. Once the device description(s) has been acquired at block 206, handheld field maintenance tool 52 will possess a comprehensive description of the capabilities and behaviors of the selected field device for which simulation is desired. At this point, method 200 continues at block 208 where handheld field maintenance tool 52 presents a user interface to the technician or user that allows the configuration of specific parameters of the simulated field device and allows the technician to specify a process variable, such as a process fluid pressure, or temperature, that is measured by the simulated field device. At block 210, handheld field maintenance tool 52 interacts with the process controller via a process communication module. Examples of such interaction include communication over a wired process communication loop or segment using wired process communication module 138. Examples of such wired process communication include the Highway Addressable Remote Transducer HART® protocol, the FOUNDATION™ Fieldbus protocol, Profibus et cetera. Alternatively, the handheld field maintenance tool may communicate over a wireless process communication protocol, such as WirelessHART using wireless process communication protocol module 121. In this sense, the handheld field maintenance tool is communicating upon a process control loop or segment to indicate the selected variable or parameter of the simulated field device set by the technician to the process controller. Certainly, embodiments of the present invention also include the ability of the handheld field maintenance tool 52 operating in device simulation mode to receive one or more responses from a process controller, or other suitable device, and react to such response(s) in accordance with the simulation as directed by technician.

Since the output of the handheld field maintenance tool represents a "false" or simulated signal, it is certainly not intended for use during live operations of a process control installation. Instead, the simulation mode is intended primarily for use as a pre-startup loop or segment verification. Additionally, because of the significant impact a false signal could have on plant operations, embodiments of the present invention includes a simulation flag 212 or other suitable data structure that is provided with the simulation communication in block 210 such that the control system is able to distinguish such signals. One example of such communication may be an additional communication packet to the control/host system that allows it to display to the operator if a device is actually a simulated device.

## Claims

1. A handheld field maintenance tool (52) comprising:
a process communication module (138) configured to communicate in accordance with a process industry communication standard;
a controller (130) coupled to the process communication module (138), the controller (130) being configured to access a device description DD relative to a selected simulated field device (104);
a user interface configured to receive a user input relative to a parameter of the simulated field device (104); and
wherein the controller (130) is configured to generate communication through the process communication module (138) to simulate the selected field device (104) based on the user input,
**characterized in that**:
the user input further comprises a specified process variable that is measured by the simulated field device (104), the simulation performed on the basis of the specified process variable, and
wherein communication through the process communication module (138) also includes a data structure which allows a control system to distinguish simulated signals.

2. The handheld field maintenance tool (52) of claim 1, wherein the process communication module (138) is a wired process communication module (138).

3. The handheld field maintenance tool of claim 1, wherein the process communication module (138) is a wireless process communication module (123).

4. The handheld field maintenance tool (52) of claim 1, and further comprising a database of device descriptions DD stored on computer readable media within the handheld field maintenance tool (52) and coupled the controller (130).

5. The handheld field maintenance tool (52) of claim 1, and further comprising at least one secondary wireless communication protocol module (121) coupled to the controller (130), wherein the controller (130) is configured to access the device description DD using the at least one secondary wireless communication protocol module (121).

6. The handheld field maintenance tool (52) of claim 1, wherein the communication generated through the process communication module (138) includes an additional communication packet to allow a host to display an indicator relative to the simulated field device (104).

7. A method of simulating a field device (104) over a process communication loop, the method comprising:
coupling a handheld field maintenance tool (52) to the loop;
selecting a field device (104) to simulate with the handheld field maintenance tool (52);
accessing a device description DD for the selected field device (104);
receiving user input relative to at least one parameter indicated by the device description DD to be a variable of the simulated field device; and
generating process communication on the loop with the handheld field maintenance tool (52) based on the device description DD and the user input,
**characterized in that**:
the user input further comprises a specified process variable that is measured by the simulated field device (104), the simulation performed on the basis of the specified process variable, and
wherein the process communication also includes a data structure which allows a control system to distinguish simulated signals.

8. The method of claim 7, wherein the handheld field maintenance tool (52) simulates a plurality of field devices (104).

9. The method of claim 7, wherein accessing the device description DD includes wirelessly communicating with a remote database to obtain the device description DD.

## Patentansprüche

1. Handgehaltenes Feldwartungswerkzeug (52), umfassend:
ein Prozesskommunikationsmodul (138), das so konfiguriert ist, dass es in Übereinstimmung mit einem Kommunikationsstandard der Prozessindustrie kommuniziert;
eine Steuerung (130), die mit dem Prozesskommunikationsmodul (138) gekoppelt ist, wobei die Steuerung (130) konfiguriert ist, um auf eine Gerätebeschreibung DD bezüglich einem ausgewählten, simulierten Feldgerät (104) zuzugreifen;
eine Benutzerschnittstelle, die so konfiguriert ist, dass sie eine Benutzereingabe bezüglich einem Parameter des simulierten Feldgeräts (104) empfängt; und
wobei die Steuerung (130) dazu konfiguriert ist, eine Kommunikation durch das Prozesskommunikationsmodul (138) zu generieren, um das ausgewählte Feldgerät (104) basierend auf der Benutzereingabe zu simulieren,
**dadurch gekennzeichnet, dass**
die Benutzereingabe ferner eine spezifizierte Prozessvariable umfasst, die von dem simulierten Feldgerät (104) gemessen wird, wobei die Simulation auf der Basis der spezifizierten Prozessvariable durchgeführt wird, und
wobei die Kommunikation durch das Prozesskommunikationsmodul (138) auch eine Datenstruktur umfasst, die es einem Steuersystem ermöglicht, simulierte Signale zu unterscheiden.

2. Handgehaltenes Feldwartungswerkzeug (52) nach Anspruch 1, wobei das Prozesskommunikationsmodul (138) ein verdrahtetes Prozesskommunikationsmodul (138) ist.

3. Handgehaltenes Feldwartungswerkzeug nach Anspruch 1, wobei das Prozesskommunikationsmodul (138) ein drahtloses Prozesskommunikationsmodul (123) ist.

4. Handgehaltenes Feldwartungswerkzeug (52) nach Anspruch 1, und ferner aufweisend eine Datenbank von Gerätebeschreibungen DD, die auf computerlesbaren Medien innerhalb des tragbaren Feldwartungswerkzeugs (52) gespeichert und mit der Steuerung (130) gekoppelt sind.

5. Handgehaltenes Feldwartungswerkzeug (52) nach Anspruch 1, und ferner aufweisend mindestens ein sekundäres drahtloses Kommunikationsprotokollmodul (121), das mit der Steuerung (130) gekoppelt ist, wobei die Steuerung (130) so konfiguriert ist, dass sie auf die Gerätebeschreibung DD unter Verwendung des mindestens einen sekundären drahtlosen Kommunikationsprotokollmoduls (121) zugreift.

6. Handgehaltenes Feldwartungswerkzeug (52) nach Anspruch 1, wobei die durch das Prozesskommunikationsmodul (138) generierte Kommunikation ein zusätzliches Kommunikationspaket enthält, um es einem Host zu ermöglichen, einen Indikator bezüglich des simulierten Feldgeräts (104) anzuzeigen.

7. Verfahren zum Simulieren eines Feldgeräts (104) über eine Prozesskommunikationsschleife, wobei das Verfahren umfasst:
Koppeln eines handgehaltenen Feldwartungswerkzeugs (52) an die Schleife;
Auswählen eines Feldgeräts (104), das mit dem handgehaltenen Feldwartungswerkzeug (52) simuliert werden soll;
Zugreifen auf eine Gerätebeschreibung DD für das ausgewählte Feldgerät (104);
Empfangen einer Benutzereingabe bezüglich mindestens einem Parameter, wobei die Gerätebeschreibung DD indiziert, dass es sich um eine Variable des simulierten Feldgeräts handelt; und Generieren einer Prozesskommunikation in der Schleife mit dem handgehaltenen Feldwartungswerkzeug (52) auf der Grundlage der Gerätebeschreibung DD und der Benutzereingabe,
**dadurch gekennzeichnet, dass**
die Benutzereingabe weiterhin eine spezifizierte Prozessvariable umfasst, die durch das simulierte Feldgerät (104) gemessen wird, wobei die Simulation auf der Basis der spezifizierten Prozessvariable durchgeführt wird, und
wobei die Prozesskommunikation auch eine Datenstruktur umfasst, die es einem Steuersystem ermöglicht, simulierte Signale zu unterscheiden.

8. Verfahren nach Anspruch 7, wobei das handgehaltene Feldwartungswerkzeug (52) eine Vielzahl von Feldgeräten (104) simuliert.

9. Verfahren nach Anspruch 7, wobei der Zugriff auf die Gerätebeschreibung DD eine drahtlose Kommunikation mit einer entfernten Datenbank einschließt, um die Gerätebeschreibung DD zu erhalten.

## Revendications

1. Outil de maintenance de terrain portatif (52) comprenant :
un module de communication de processus (138) configuré pour communiquer conformément à un référentiel de communication d'industrie de processus ;
un dispositif de commande (130) couplé au module de communication de processus (138), le dispositif de commande (130) étant configuré pour accéder à une description de dispositif DD relative à un dispositif de terrain (104) simulé sélectionné ;
une interface utilisateur configurée pour recevoir une entrée d'utilisateur relative à un paramètre du dispositif de terrain (104) simulé ; et
sachant que le dispositif de commande (130) est configuré pour générer une communication via le module de communication de processus (138) pour simuler le dispositif de terrain (104) sélectionné sur la base de l'entrée d'utilisateur,
**caractérisé en ce que** :
l'entrée d'utilisateur comprend en outre une variable de processus spécifiée qui est mesurée par le dispositif de terrain (104) simulé, la simulation étant effectuée sur la base de la variable de processus spécifiée, et
sachant qu'une communication via le module de communication de processus (138) inclut également une structure de données qui permet à un système de commande de distinguer des signaux simulés.

2. L'outil de maintenance de terrain portatif (52) de la revendication 1, sachant que le module de communication de processus (138) est un module de communication de processus câblé (138).

3. L'outil de maintenance de terrain portatif de la revendication 1, sachant que le module de communication de processus (138) est un module de communication de processus sans fil (123).

4. L'outil de maintenance de terrain portatif (52) de la revendication 1, et comprenant en outre une base de données de descriptions de dispositif DD stockées sur des supports lisibles par ordinateur à l'intérieur de l'outil de maintenance de terrai portatif (52) et couplés au dispositif de commande (130).

5. L'outil de maintenance de terrain portatif (52) de la revendication 1, et comprenant en outre au moins un module de protocole de communication sans fil secondaire (121) couplé au dispositif de commande (130), sachant que le dispositif de commande (130) est configuré pour accéder à la description de dispositif DD moyennant l'au moins un module de protocole de communication sans fil secondaire (121).

6. L'outil de maintenance de terrain portatif (52) de la revendication 1, sachant que la communication générée via le module de communication de processus (138) inclut un paquet de communication supplémentaire pour permettre à un hôte d'afficher un indicateur relatif au dispositif de terrain (104) simulé.

7. Procédé de simulation d'un dispositif de terrain (104) via une boucle de communication de processus, le procédé comprenant :
le couplage d'un outil de maintenance de terrain portatif (52) à la boucle ;
la sélection d'un dispositif de terrain (104) pour simuler avec l'outil de maintenance de terrain portatif (52) ;
le fait d'accéder à une description de dispositif DD pour le dispositif de terrain (104) sélectionné ;
la réception d'une entrée d'utilisateur relative à au moins un paramètre indiqué par la description de dispositif DD pour être une variable du dispositif de terrain simulé ; et
la génération d'une communication de processus sur la boucle avec l'outil de maintenance de terrain portatif (52) sur la base de la description de dispositif DD et de l'entrée d'utilisateur,
**caractérisé en ce que**
l'entrée d'utilisateur comprend en outre une variable de processus spécifiée qui est mesurée par le dispositif de terrain (104) simulé, la simulation étant effectuée sur la base de la variable de processus spécifiée, et
sachant que la communication de processus inclut également une structure de données qui permet à un système de commande de distinguer des signaux simulés.

8. Le procédé de la revendication 7, sachant que l'outil de maintenance de terrain portatif (52) simule une pluralité de dispositifs de terrain (104).

9. Le procédé de la revendication 7, sachant que le fait d'accéder à la description de dispositif DD inclut le fait de communiquer sans fil avec une base de données distante pour obtenir la description de dispositif DD.
